# EUROPEAN PATENT SPECIFICATION

(11) **EP 4 554 658 B1**
(45) Date of publication and mention of the grant of the patent: **09.12.2026**
(21) Application number: 23786707.2
(22) Date of filing: 28.09.2023
(51) Int. Cl.: A61N 1/04, A61N 1/36, A61N 1/40

(54) **SINGLE WIRE TEMPERATURE MEASUREMENT SOLUTION FOR A TTFIELD APPLICATION SYSTEM**
EINZELDRAHT-TEMPERATURMESSLÖSUNG FÜR EIN TTFIELD-ANWENDUNGSSYSTEM
SOLUTION DE MESURE DE TEMPÉRATURE À FIL UNIQUE POUR SYSTÈME D'APPLICATION DE CHAMPS DE TRAITEMENT DE TUMEUR

(30) Priority: 30.09.2022 US 202263377820 P
(43) Date of publication of application: 21.05.2025
(73) Proprietor: NOVOCURE GMBH, 6340 Baar (CH)
(72) Inventor: WASSERMAN, Yoram, 31905 Haifia (IL)
(74) Representative: Carpmaels & Ransford LLP
(86) International application number: PCT/IB2023/059723
(87) International publication number: WO 2024/069539

(56) References cited:
- CN-A- 101 988 856
- US-A1- 2018 050 200
- US-A1- 2021 196 348
- US-B2- 8 764 675

## Description

The present application relates to temperature measurement in a transducer array.

### BACKGROUND OF THE INVENTION

Tumor Treating Fields (TTFields or TTFs) are low intensity (e.g., 1-3 V/cm) alternating electric fields within the intermediate frequency range (e.g., 50 kHz to 1 MHz, such as 50-500 kHz) that target solid tumors by disrupting mitosis. This non-invasive treatment targets solid tumors and is described, for example, in US Patent Nos. 7,016,725; 7,089,054; 7,333,852; 7,565,205; 8,244,345; 8,715,203; 8,764,675; 10,188,851; and 10,441,776. TTFields are typically delivered through two pairs of transducer arrays that generate perpendicular fields within the treated tumor; the transducer arrays that make up each of these pairs are positioned on opposite sides of the body part that is being treated. More specifically, for the OPTUNE^{®} system, one pair of electrodes of the transducer array is located to the left and right (LR) of the tumor, and the other pair of electrodes of the transducer array is located anterior and posterior (AP) to the tumor. TTFields are approved for the treatment of glioblastoma multiforme (GBM), and may be delivered, for example, via the OPTUNE^{®} system (Novocure Limited, St. Helier, Jersey), which includes transducer arrays placed on the patient's shaved head. More recently, TTFields therapy has been approved as a combination therapy with chemotherapy for malignant pleural mesothelioma (MPM), and may find use in treating tumors in other parts of the body.

The device is intended to be continuously worn by the patient for 2-4 days before removal for hygienic care and re-shaving (if necessary), followed by reapplication with a new set of arrays. Because patients use the device and go about their daily activities, the device may be used for an extended period of time during which the transducer array may generate heat while activated. In order to ensure the patient is comfortable while wearing the transducer arrays, temperature sensors are placed within the arrays to monitor temperatures at the transducer array - skin interface.

The amplitude of the alternating current that is delivered via the transducer arrays is controlled so that skin temperature (as measured on the skin below the transducer arrays) does not exceed a comfortability threshold. In the existing Optune^{®} system, each array includes 8 temperature sensors.

The temperature sensors in each transducer array are connected via wires to a controller where the temperatures from all temperature sensors are measured and analog-to-digital converted into digital values for each temperature sensor. The controller uses the temperature measurements to control the current to be delivered via each pair of arrays in order to maintain temperatures below the comfortability threshold on the patient's skin. The current itself is delivered to each array via an additional wire (i.e., one wire for each array) that runs from the field generator to the transducer array.

In the existing Optune^{®} system there are four long 10-wire cables (each of which runs between a respective transducer array and the controller) that runs between the field generator and the controller. Each of the 10-wire cables has 8 wires for carrying signals from the eight temperature sensors, 1 wire for the common of all eight temperature sensors, plus 1 wire for providing the TTFields signal to the transducer array.

Attaching temperature sensors and transducer arrays to a patient is cumbersome due to the quantity of wires and combined weight of the transducer arrays and wiring. Due to the number of wires and increased weight, movement of a patient may inadvertently loosen the transducer arrays and cause the patient to become uncomfortable due to the weight. As such, there is a need for a temperature measurement solution using fewer wires. It is to such systems and methods of producing and using the same, that the present disclosure is directed.

US-A-8764675 discloses an electrode for applying electric fields to a patient which includes a plurality of ceramic elements, with temperature sensors being provided to at least some of the ceramic elements.

CN-A-101988856 discloses a temperature sensor which includes a plurality of temperature measurement parts connected in series by a signal line.

### SUMMARY OF THE INVENTION

In one aspect the present invention provides a transducer array according to claim 1.

Embodiments of the invention are disclosed in the dependent claims.

The details of one or more implementations of the subject matter described in this specification are set forth in the accompanying drawings and the description below. Other aspects, features and advantages of the subject matter will become apparent from the description, the drawings, and the claims.

### BRIEF DESCRIPTION OF THE DRAWINGS

The accompanying drawings, which are incorporated in and constitute a part of this specification, illustrate one or more implementations described herein and, together with the description, explain these implementations. The drawings are not intended to be drawn to scale, and certain features and certain views of the figures may be shown exaggerated, to scale or in schematic in the interest of clarity and conciseness. Not every component may be labeled in every drawing. Like reference numerals in the figures may represent and refer to the same or similar element or function. In the drawings:
FIG. 1 is an exemplary embodiment of a schematic diagram of electrodes as applied to living tissue;
FIG. 2 is an exemplary embodiment of an electronic device configured to generate a TTField constructed in accordance with the present disclosure;
FIG. 3 is a block diagram of an exemplary embodiment of a transducer array constructed in accordance with the present disclosure;
FIG. 4 is a capacitive reactance graph of reactance to frequency showing the reactance of two RC circuits being subjected to varying frequencies;
FIG. 5A is a schematic diagram of a temperature sensing circuit constructed in accordance with the present disclosure;
FIG. 5B is a schematic diagram of another embodiment of a temperature sensing circuit constructed in accordance with the present disclosure;
FIG. 6 is a process flow diagram of a non-claimed method for determining temperatures of electrodes within the transducer array in accordance with the present disclosure;

### DETAILED DESCRIPTION

Before explaining at least one embodiment of the inventive concept(s) in detail by way of exemplary language and results, it is to be understood that the inventive concept(s) is not limited in its application to the details of construction and the arrangement of the components set forth in the following description. The inventive concept(s) is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Headings are provided for convenience only and are not to be construed to limit the disclosure in any manner. Embodiments illustrated under any heading or in any portion of the disclosure may be combined with embodiments illustrated under the same or any other heading or other portion of the disclosure. Any combination of the elements described herein in all possible variations thereof is encompassed by the disclosure unless otherwise indicated herein or otherwise clearly contradicted by context.

Unless otherwise required by context, singular terms shall include pluralities and plural terms shall include the singular.

All of the compositions, assemblies, systems, kits, and/or methods disclosed herein can be made and executed without undue experimentation in light of the present disclosure. Where a method claim does not specifically state in the claims or description that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of embodiments described in the specification.

The use of the term "a" or "an" when used in conjunction with the term "comprising" in the claims and/or the specification may mean "one," but is also consistent with the meaning of "one or more," "at least one," and "one or more than one." The term "plurality" refers to "two or more."

In addition, the use of the term "at least one of X, Y, and Z" will be understood to include X alone, Y alone, and Z alone, as well as any combination of X, Y, and Z. The use of ordinal number terminology (e.g., "first," "second," "third," "fourth," etc.) is solely for the purpose of differentiating between two or more items and is not meant to imply any sequence or order or importance to one item over another or any order of addition, for example.

The use of the term "or" in the claims is used to mean an inclusive "and/or" unless explicitly indicated to refer to alternatives only or unless the alternatives are mutually exclusive.

Circuitry, as used herein, may be analog and/or digital components, or one or more suitably programmed processors (e.g., microprocessors) and associated hardware and software, or hardwired logic. Also, "components" may perform one or more functions. The term "component," may include hardware, such as a processor (e.g., microprocessor), an application specific integrated circuit (ASIC), a field programmable gate array (FPGA), a combination of hardware and software, and/or the like. The term "processor" as used herein means a single processor or multiple processors working independently or together to collectively perform a task. The processor may communicate with a non-transitory computer-readable medium storing computer-executable instructions that when executed by the processor causes the processor to perform a specified function. Exemplary non-transitory computer-readable mediums may include a non-volatile memory, a random access memory (RAM), a read only memory (ROM), a CD-ROM, a hard drive, a solid-state drive, a flash drive, a memory card, a DVD-ROM, a BluRay Disk, a laser disk, a magnetic disk, an optical drive, combinations thereof, and/or the like.

As used herein, the term TTField (TTFields, or TTF(s)) refers to low intensity (e.g., 1-4 V/cm) alternating electric fields of medium frequencies (about 50 kHz - 1 MHz, and more preferably from about 50 kHz - 500 kHz) that when applied to a conductive medium, such as a human body, via electrodes may be used, for example, to treat tumors as described in US Patent Nos. 7,016,725, 7,089,054, 7,333,852, 7,565,205, 7,805,201, and 8,244,345 by Palti and in a publication by Kirson (see Eilon D. Kirson, et al., Disruption of Cancer Cell Replication by Alternating Electric Fields, Cancer Res. 2004 64:3288-3295). TTFields have been shown to have the capability to specifically affect cancer cells and serve, among other uses, for treating cancer. TTFields therapy is an approved mono-treatment for recurrent glioblastoma (GBM), and an approved combination therapy with chemotherapy for newly diagnosed GBM patients.

As used herein, the term TTSignal is an electrical signal that, when received by electrodes applied to a conductive medium, such as a human body, causes the electrodes to generate the TTField described above. The TTSignal is often an AC electrical signal.

As used herein, all numerical values or ranges include fractions of the values and integers within such ranges and fractions of the integers within such ranges unless the context clearly indicates otherwise. The numerical ranges specified herein includes the endpoints, and all values, sub-ranges of values within the range, and fractions of the values and integers within said range. Thus, any two values within the range of 1 mm to 10 m, for example, can be used to set a lower and an upper boundary of a range in accordance with the embodiments of the present disclosure.

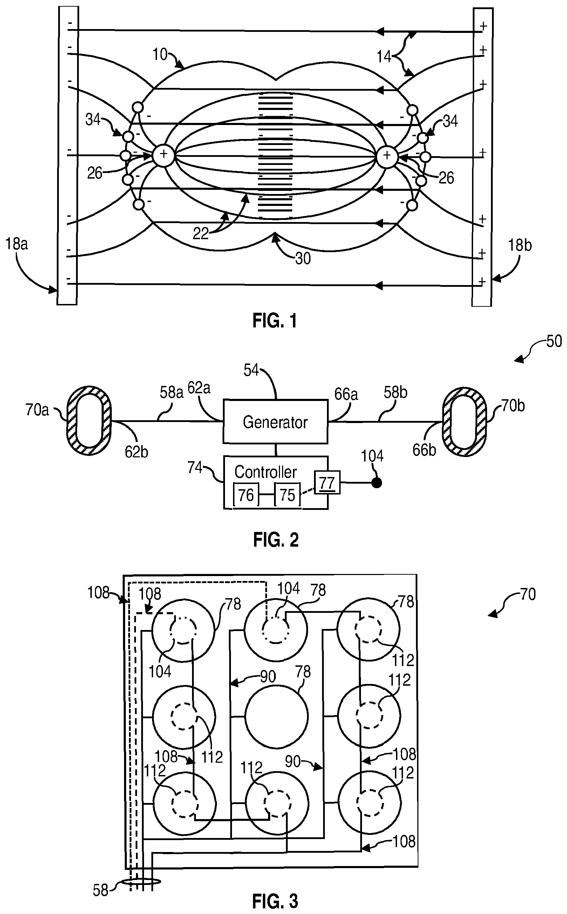

Referring now to the drawings and in particular to FIG. 1, shown therein is an exemplary embodiment of a dividing cell 10, under the influence of external TTFields, generally indicated as lines 14, generated by a first electrode 18a having a negative charge and a second electrode 18b having a positive charge. Further shown are microtubules 22 that are known to have a very strong dipole moment. This strong polarization makes the microtubules 22, as well as other polar macromolecules and especially those that have a specific orientation within the cell 10 or its surroundings, susceptible to electric fields. The microtubules 22 positive charges are located at two centrioles 26 while two sets of negative poles are at a center 30 of the dividing cell 10 and point of attachment 34 of the microtubules 22 to the cell membrane. The locations of the charges form sets of double dipoles and therefore are susceptible to electric fields of differing directions. In one embodiment, the cells go through electroporation, that is, DNA or chromosomes are introduced into the cells using a pulse of electricity to briefly open pores in the cell membranes.

Turning now to FIG. 2, the TTFields described above that have been found to advantageously destroy tumor cells may be generated by an electronic apparatus 50. FIG. 2 is a simple schematic diagram of the electronic apparatus 50 illustrating major components thereof. The electronic apparatus 50 includes an electric field generator 54 and a pair of conductive leads 58, including first conductive lead 58a and second conductive lead 58b. The first conductive lead 58a includes a first end 62a and a second end 62b. The second conductive lead 58b includes a first end 66a and a second end 66b. The first end 62a of the first conductive lead 58a is conductively attached to the electric field generator 54 and the first end 66a of the second conductive lead 58b is conductively attached to the electric field generator 54.

The electric field generator 54 is configured to supply power and generate desirable electric signals (TTSignals) in the shape of waveforms or trains of pulses as an output. The second end 62b of the first conductive lead 58a is connected to a transducer array 70a and the second end 66b of the second conductive lead 58b is connected to a transducer array 70b. Both of the transducer array 70a and the transducer array 70b are supplied with the electric signals (e.g., TTSignals, wave forms). The transducer array 70a and the transducer array 70b, being supplied with the electric signals, causes an electrical current to flow between the transducer array 70a and the transducer array 70b. The electrical current generates an electric field (i.e., TTField), having a frequency and an amplitude, to be generated between the transducer array 70a and the transducer array 70b.

While the electronic apparatus 50 shown in FIG. 2 comprises only two transducer arrays 70 (i.e., the transducer array 70a and the transducer array 70b), in some embodiments, the electronic apparatus 50 may comprise more than two transducer arrays 70.

The electric field generator 54 generates an alternating voltage wave form (i.e., TTSignal) at frequencies in the range from about 50 kHz to about 1 MHz (preferably from about 100 kHz to about 500 kHz). The required voltages are such that an electric field intensity in tissue within the treatment area is in the range of about 0.1 V/cm to about 10 V/cm. To achieve this electric field intensity, the potential difference between the two conductors 18 (e.g., the electrode element 78 in FIG. 3) in each of the transducer array 70a or the transducer array 70b is determined by the relative impedances of the system components, e.g., a fraction of the electric field on each component is given by that component's impedance divided by a total circuit impedance.

In certain particular (but non-limiting) embodiments, the transducer array 70a and the transducer array 70b generate an alternating electric current and field within a target region of a patient. The target region typically comprises at least one tumor, and the generation of the alternating electric current and field selectively destroys and/or inhibits growth of the tumor. The alternating electric current and field may be generated at any frequency that selectively destroys or inhibits growth of the tumor, such as at any frequency of a TTField.

In certain particular (but non-limiting) embodiments, the alternating electric current and field may be imposed at two or more different frequencies. When two or more frequencies are present, each frequency is selected from any of the above-referenced values, or a range formed from any of the above-referenced values, or a range that combines two integers that fall between two of the above-referenced values.

In order to optimize the electric field (i.e., TTField) distribution, the transducer array 70a and the transducer array 70b (pair of transducer arrays 70) may be configured differently depending upon the application in which the pair of transducer arrays 70 are to be used. The pair of transducer arrays 70, as described herein, are externally applied to a patient, that is, are generally applied to the patient's skin, in order to apply the electric current, and electric field (TTField) thereby generating current within the patient's tissue. Generally, the pair of transducer arrays 70 are placed on the patient's skin by a user such that the electric field is generated across patient tissue within a treatment area. TTFields that are applied externally can be of a local type or widely distributed type, for example, the treatment of skin tumors and treatment of lesions close to the skin surface.

In one embodiment, the user may be a medical professional, such as a doctor, nurse, therapist, or other person acting under the instruction of a doctor, nurse, or therapist. In another embodiment, the user may be the patient, that is, the patient (and/or a helper) may place the transducer array 70a and the transducer array 70b on the patient's treatment area.

According to another exemplary embodiment, the electronic apparatus 50 includes a controller 74. In one embodiment, the controller 74 comprises circuitry configured to control the output of the electric field generator 54, for example, to set the output at the maximal value that does not cause excessive heating of the treatment area. The controller 74 may issue a warning, or the like, when a temperature of the treatment area (as sensed by a temperature sensor 104, discussed in more detail below) exceeds a preset limit. The temperature sensor 104 may be mechanically connected to and/or otherwise associated with the transducer array 70a and/or the transducer array 70b so as to sense the temperature of the treatment area at either one or both of the transducer array 70a or the transducer array 70b as described below in more detail.

In one embodiment, the controller 74 may turn off, or decrease power of the TTSignal generated by the electric field generator 54, if a temperature sensed by the temperature sensor meets or exceeds a comfortability threshold. In one embodiment, the comfortability threshold is the temperature at which a patient would be made uncomfortable while using the transducer array 70a and the transducer array 70b. For example, the comfortability threshold may be a temperature at or about 40 degrees Celsius. In one embodiment, the comfortability threshold is a temperature of between about 39 degrees Celsius and 42 degrees Celsius, or a specific selected temperature between about 39 degrees Celsius and 42 degrees Celsius.

In one embodiment, the controller 74 includes circuitry including a processor 75 and a memory 76. The memory 76 may be a non-transitory computer-readable medium (e.g., a random access memory, and/or a read only memory) storing computer executable instructions that when executed by the processor 75 causes the processor 75 to perform one or more function. The processor 75 may be in communication with the one or more temperature sensor 104 and/or in communication with circuitry, such as an analog to digital converter, a multimeter, an ohmmeter, a voltmeter, and/or an ammeter.

The conductive leads 58 are isolated conductors with a flexible metal shield, preferably grounded thereby preventing spread of any electric field generated by the conductive leads 58. The transducer array 70a and the transducer array 70b may have specific shapes and positioning so as to generate the TTField of a desired configuration, direction, and intensity at the treatment area and only at that treatment area so as to focus the treatment.

The specifications of the electronic apparatus 50 as a whole and its individual components are largely influenced by the fact that at the frequency of the TTFields, living systems behave according to their "Ohmic", rather than their dielectric properties.

In one embodiment, the controller 74 further includes measurement circuitry 77. For example, measuring a resistance of the temperature sensor 104 may include the processor 75 communicating with and causing the measurement circuitry 77 to measure the resistance. The measurement circuitry 77 may include, for example, an ohmmeter, an ammeter, a voltmeter, and a multimeter. In one embodiment, the measurement circuitry 77 further includes an analog to digital converter and may convert the resistance measurement into a digital signal provided to the processor 75.

Referring now to FIG. 3, shown therein is a diagram of an exemplary embodiment of the transducer array 70 constructed in accordance with the present disclosure. The transducer array 70 includes one or more electrode element 78. As shown in FIG. 3, each transducer array 70 is configured as a set of one or more electrode elements 78. Transducer arrays 70 may utilize electrode elements 78 that are capacitively coupled with the patient.

In the example shown in FIG. 3, the transducer array 70 is configured as multiple electrode elements 78 (for example, about 2 cm in diameter) that are interconnected via flex wires 90 (and connected to the electric field generator 54 via the conductive lead 58).

In one embodiment, the transducer array 70 further includes a plurality of temperature sensors 104 associated with one or more of the electrode elements 78. As shown in FIG. 3, the transducer array 70 includes eight temperature sensors 104 associated with eight of the nine electrode elements 78; however, a temperature sensor 104 may be associated with more or fewer of the electrode element 78 of the transducer array 70. Each of the temperature sensor 104 is connected to at least one other temperature sensor 104 via a sensor wire 108.

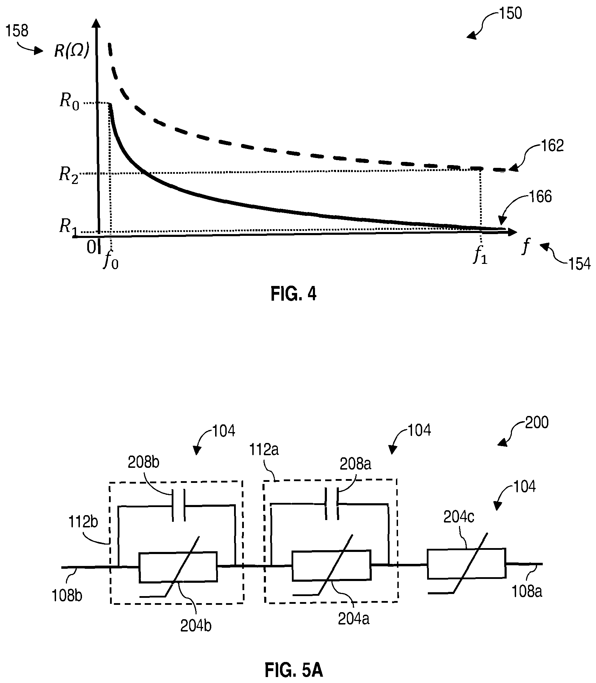

In one embodiment, the transducer array 70 further includes one or more RC circuit 112. Each RC circuit 112, shown in FIG. 5A and described in more detail below, may be connected to at least one other of a temperature sensor 104 or an RC circuit 112.

Referring now to FIG. 4, shown therein is a diagram of an exemplary embodiment of a capacitive reactance graph 150 in accordance with the present disclosure. The capacitive reactance graph 150 is shown with a first axis 154 denoting a frequency and a second axis 158 denoting a resultant resistance in a logarithmic scale. Further shown is a first plot 162 illustrating the capacitive reactance for a first capacitor as a frequency of an applied current is increased and having a first capacitance and a second plot 166 illustrating the capacitive reactance for a second capacitor as the frequency of the applied current is increased and having a second capacitance, where the second capacitance is 10x the first capacitance.

As shown in FIG. 4, as the frequency increases, the resultant impedance of the capacitor decreases. For example, a first resultant reactance R₀ of the second capacitor at a first frequency f₀ is larger than a second resultant reactance R₁ of the second capacitor at a second frequency f₁ and is quite near a 0 Ω reactance, e.g., nearly a short-circuit. At that same second frequency f₁, however, the first capacitor has a third resultant reactance R₂ greater than the second resultant reactance R₁. As shown in FIG. 4, therefore, the resultant reactance of a capacitor decreases, and approaches zero (0) Ω, as the frequency of the applied current increases.

Referring now to FIG. 5A, shown therein is a circuit diagram of an exemplary embodiment of a temperature sensing circuit 200 constructed in accordance with the present disclosure. The temperature sensing circuit 200 generally comprises a third thermistor 204c, i.e., a temperature sensor 104, electrically connected to a first sensor wire 108a and disposed in series with at least one RC circuit 112, the at least one RC circuit 112 is further connected to a second sensor wire 108b opposite the first sensor wire 108a. The third thermistor 204c and each of the RC circuits 112a and 112b are in thermal communication with (e.g., attached to) an electrode element 78 (not shown in FIG. 5A). It should be noted that each of the third thermistor 204c, the RC circuit 112a, and the second RC circuit 112b may be arranged in series within the temperature sensing circuit 200 in any order, and not necessarily in the order illustrated in FIG. 5A.

As shown in FIG. 5A, the temperature sensing circuit 200 comprises a third thermistor 204c electrically connected to the first sensor wire 108a and electrically disposed in series with a first RC circuit 112a and a second RC circuit 112b, the second RC circuit 112b is further connected to a second sensor wire 108b. The temperature sensing circuit 200 may be provided with additional RC circuits 112 for measuring the temperature of other electrode elements 78 in series with the other RC circuits 112a and 112b and the third thermistor 204c.

In one embodiment, each thermistor 204 is a 10kΩ thermistor (e.g., the thermistor 204 has a resistance of 10kΩ at 20°C and a resistance of about 5k Ω at 40°C) such that a resistance, *R,* of the thermistor 204 is strongly related to a temperature of the thermistor, that is, each thermistor 204 is a variable resistor having a resistance, R, that varies with temperature wherein the thermistor 204 has known properties of corresponding resistance and temperature. For example, a temperature of the thermistor 204 may be determined by routing a known current through the thermistor 204 and measuring a voltage across the thermistor 204. With the current (*I*) and the voltage (V) known, a resistance, R, of the thermistor 204 may be determined using Ohm's law, $R = \frac{V}{I}$. The resistance, R, may then be converted to a temperature based on known properties of the thermistor 204. In one embodiment, the thermistor 204 is a negative temperature coefficient thermistor or a negative temperature coefficient variable resistor.

In one embodiment, each RC circuit 112 comprises a thermistor 204 and a capacitor 208 in parallel with the thermistor. As described above, the thermistor 204 may be a 10kΩ thermistor, although other values can be used. The capacitor 208 of each RC circuit 112 may be selected to have a capacitance less than a capacitance of the next smallest capacitor in the temperature sensing circuit 200. Thus, as more RC circuits 112 are added to the temperature sensing circuit 200, the capacitance of the capacitor of each newly added RC circuit 112 is less than the capacitance of the capacitors in any other RC circuit 122 in the temperature sensing circuit 200. In one embodiment, the capacitance of each capacitor is selected from within a range of between about 10 mf (i.e., 10 millifarad) and about 1 pf (i.e., 1 picofarad). In some embodiments, the difference in capacitance between the capacitor 208 of each RC circuit 112 is within a range of one order of magnitude to four orders of magnitude. In some embodiments, the difference in capacitance between the capacitor 208 of each RC circuit 112 is within a range of 100 to 10,000 times.

Referring again to FIG. 5A, the temperature sensing circuit 200 comprises the third thermistor 204c (without a capacitor in parallel), the first RC circuit 112a, and the second RC circuit 112b, all wired in series. The temperature sensing circuit 200 further includes the first sensor wire 108a and the second sensor wire 108b, each electrically coupled to the temperature sensing circuit 200.

In one embodiment, the first RC circuit 112a comprises a first capacitor 208a electrically disposed in parallel with a first thermistor 204a and the second RC circuit 112b comprises a second capacitor 208b electrically disposed in parallel with a second thermistor 204b. The first capacitor 208a is selected such that a first capacitance of the first capacitor 208a is greater than a second capacitance of the second capacitor 208b. In some embodiments, the second capacitance of the second capacitor 208b is selected to be within a range of at least 1/10 (one-tenth) the first capacitance of the first capacitor 208a to 1/100,000 (one-hundred-thousandth) the first capacitance of the first capacitor 208a. In some embodiments, the second capacitance of the second capacitor 208b is selected to be less than 1/1,000,000 (one-millionth) the first capacitance of the first capacitor 208a.

A user may measure an impedance across the temperature sensing circuit 200, e.g., by attaching a first probe of an ohmmeter to the first sensor wire 108a and a second probe of the ohmmeter to the second sensor wire 108b. By applying a first sensing signal having a known current and a first frequency, a total impedance R_{T} may be measured (e.g., a resistance Rₓ of the third thermistor 204c plus a resistance R_{y} of the first thermistor 204a plus a resistance R_{z} of the second thermistor 204b). The user may then apply a second sensing signal having the known current and a second frequency sufficient to cause a first reactance of the first capacitor 208a to approach zero while also insufficient to cause a second reactance of the second capacitor 208b to approach zero, thereby effectively shorting the temperature sensing circuit 200 across the first thermistor 204a, to measure a second impedance Rₐ (e.g., the resistance R_{z} of the second thermistor 204b plus the resistance Rₓ of the third thermistor 204c). In some embodiments, the second sensing signal has the second frequency in a range of between one order of magnitude and four orders of magnitude greater than the first frequency. Finally, the user may then apply a third sensing signal having the known current and a third frequency sufficient to cause the first reactance of the first capacitor 208a to approach zero while also sufficient to cause the second reactance of the second capacitor 208b to approach zero, thereby effectively shorting the temperature sensing circuit 200 across the first thermistor 204a and the second thermistor 204b, to measure a third impedance R_{b} (e.g., the resistance Rₓ of the third thermistor 204c). Thus, the resistance Rₓ of the third thermistor 204c is measured as the third impedance R_{b}, the resistance R_{y} of the first thermistor 204a is calculated, such that *R_{y} = R_{T}* - *Rₐ*, e.g., as the total impedance R_{T} minus the second impedance Rₐ, and the resistance R_{z} of the second thermistor 204b is calculated, such that *R_{z}* = *Rₐ* - *R_{b}*, e.g., as the second impedance Rₐ minus the third impedance R_{b}.

In some embodiments, then, the first capacitor 208a and the second capacitor 208b may be selected such that the second frequency supplied to the first capacitor 208a is sufficient to cause the first reactance of the first capacitor 208a to approach zero, while the second capacitor 208b is selected such that the second frequency supplied to the second capacitor 208b is insufficient to cause the second reactance of the second capacitor 208b to approach zero.

In some embodiments, the third thermistor 204c may further include a third capacitor electrically disposed in parallel with the third thermistor 204c thereby forming a third RC circuit 112. As described below in more detail, having the third capacitor in parallel with the third thermistor 204c is not necessary, however, such an arrangement would require that each sensing signal have a frequency sufficient to effectively "short" across at least one capacitor. When the third capacitor is not present, that is when the third thermistor 204c does not include a capacitor disposed in parallel, one sensing signal applied to the temperature sensing circuit 200 does not require a frequency sufficient to effectively "short" across any capacitor, e.g., does not require a frequency sufficient to cause any capacitor to have a reactance approaching zero.

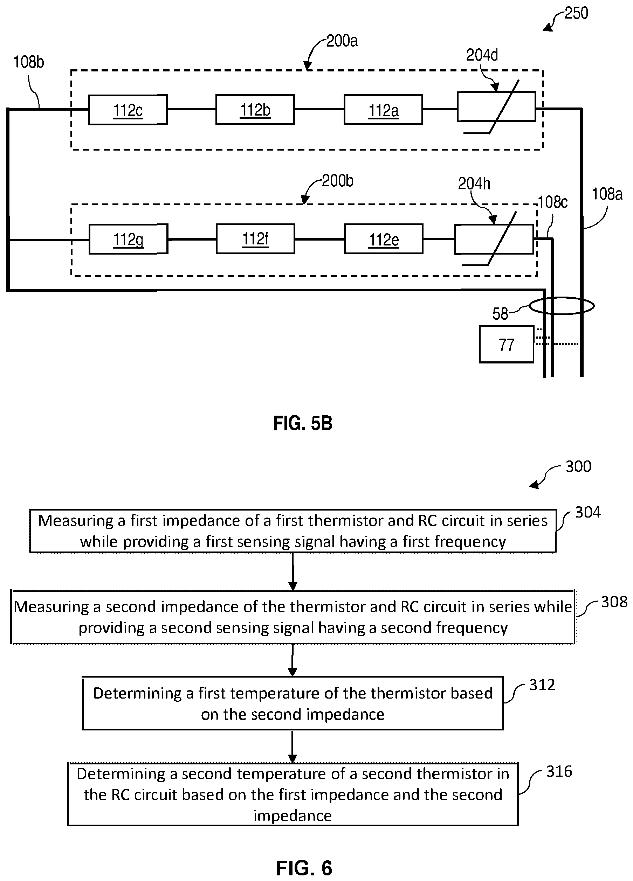

Referring now to FIG. 5B, shown therein is a circuit diagram of an exemplary embodiment of a complex temperature sensing circuit 250 constructed in accordance with the present disclosure. Generally, the complex temperature sensing circuit 250 is constructed of two or more temperature sensing circuits 200, shown in FIG. 5B as a first temperature sensing circuit 200a and a second temperature sensing circuit 200b. Each of the first temperature sensing circuit 200a and the second temperature sensing circuit 200b is constructed similar to the temperature sensing circuit 200 shown in FIG. 5A with the exception that each of the first temperature sensing circuit 200a and the second temperature sensing circuit 200b includes a third RC circuit 112.

As shown in FIG. 5B, the first sensor wire 108a is connected to the first temperature sensing circuit 200a and is operable to carry a first sensing signal from the controller 74 to the first temperature sensing circuit 200a. The second sensor wire 108b is connected to the first temperature sensing circuit 200a and is operable to carry the first sensing signal from the first temperature sensing circuit 200a to the controller 74. Further shown is a third sensor wire 108c, operable to carry a second sensing signal from the controller 74 to the second temperature sensing circuit 200b. The second temperature sensing circuit 200b is connected to the second sensor wire 108b wherein the second sensor wire 108b is further operable to carry the second sensing signal from the second temperature sensing circuit 200b back to the controller 74. By sharing the second sensor wire 108b, the complex temperature sensing circuit 250 may further reduce the number of wires required within the conductive lead 58. In the embodiment shown, the complex temperature sensing circuit 250 includes three wires, but the controller 74 is operable to measure the temperature of eight electrode elements 78 with each electrode element 78 in thermal communication with one of the RC circuits 112a-c, the RC circuits 112e-g, and the thermistors 204d and 204h. While the complex temperature sensing circuit 250 is shown with only two temperature sensing circuits 200, it should be understood that additional complex temperature sensing circuits 250 may be included.

In operation, the complex temperature sensing circuit 250 may receive, e.g., from the controller 74, a plurality of first sensing signals along the first sensor wire 108a and the second sensor wire 108b to measure a temperature of each thermistor 204 of the first temperature sensing circuit 200a and may receive, e.g., from the controller 74, a plurality of second sensing signals along the third sensor wire 108c and the second sensor wire 108b to measure a temperature of each thermistor 204 of the second temperature sensing circuit 200b. Because the first temperature sensing circuit 200a and the second temperature sensing circuit 200b are not in series with each other, the first sensing signals and the second sensing signals do not require frequencies offset from one another.

Alternatively, in another embodiment, the controller 74 may provide the first sensing signal along the first sensor wire 108a at a first time and the first sensing signal along the third sensor wire 108c at a second time to poll the complex temperature sensing circuit 250 and determine a resistance for each thermistor 204. For example, the controller 74 may supply the first sensing signal having a first frequency along the first sensor wire 108a at a first time, measure a first impedance between the first sensor wire 108a and the second sensor wire 108b, provide the first sensing signal having the first frequency along the third sensor wire 108c at a second time, and measure a second impedance between the third sensor wire 108c and the second sensor wire 108b. The controller 74 may then supply the first sensing signal having a second frequency along the first sensor wire 108a at a third time, measure a third impedance between the first sensor wire 108a and the second sensor wire 108b, provide the first sensing signal having the second frequency along the third sensor wire 108c at a fourth time, and measure a fourth impedance between the third sensor wire 108c and the second sensor wire 108b. The controller 74 may supply the first sensing signal having a third frequency along the first sensor wire 108a at a fifth time, measure a fifth impedance between the first sensor wire 108a and the second sensor wire 108b, provide the first sensing signal having the third frequency along the third sensor wire 108c at a sixth time, and measure a sixth impedance between the third sensor wire 108c and the second sensor wire 108b. Finally, the controller 74 may supply the first sensing signal having a fourth frequency along the first sensor wire 108a at a seventh time, measure a seventh impedance between the first sensor wire 108a and the second sensor wire 108b, provide the first sensing signal having the fourth frequency along the third sensor wire 108c at an eighth time, and measure an eighth impedance between the third sensor wire 108c and the second sensor wire 108b.

In this embodiment, the first impedance may be, for example, a combined resistance of the thermistor 204d and impedance of the RC circuits 112a-c, while the second impedance may be a combined resistance of the thermistor 204h and impedance of the RC circuits 112e-g, the third impedance may be a combined resistance of the thermistor 204d and impedance of two of the RC circuits 112a-c (e.g., the RC circuits 112a-b), the fourth impedance may be a combined resistance of the thermistor 204h and-impedance of two of the RC circuits 112e-g (e.g., the RC circuits 112e-f), the fifth impedance may be a combined resistance of the thermistor 204d and impedance of one of the RC circuits 112a-c (e.g., the RC circuit 112a), the sixth impedance may be a combined resistance of the thermistor 204h and impedance of one of the RC circuits 112eg (e.g., the RC circuit 112e), the seventh impedance may be a resistance of the thermistor 204d and the eighth impedance may be a resistance of the thermistor 204h.

In this embodiment, when the same frequency is used for both temperature sensing circuit 200a-b of the complex temperature sensing circuit 250, at least one RC circuit 112 from each temperature sensing circuit 200 may have a capacitor 208 with a capacitance within 15% of each other. For example, the RC circuit 112a and the RC circuit 112e may each have a capacitor with a capacitance within about 15% of each other, as do the second RC circuit 112b and the RC circuit 112f, as well as the RC circuit 112c and the RC circuit 112g. In this way, the frequency applied to a particular one of the temperature sensing circuits 200 is similarly effective when applied to the other of the temperature sensing circuits 200.

Referring now to FIG. 6, shown therein is a process flow diagram of an exemplary embodiment of a sensing process 300 constructed in accordance with the present disclosure. The sensing process 300 generally comprises the steps of: measuring a first impedance of a temperature sensing circuit (step 304); measuring a second impedance of the temperature sensing circuit (step 308); determining a first temperature of the thermistor (step 312); and determining a second temperature of the RC circuit (step 316). The sensing process 300 may be stored as a series of computer-executable instructions in the memory 76 and may be executed by the processor 75.

In one embodiment, the sensing process 300 is performed more than once and may be performed periodically. For example, in one embodiment, as soon as the sensing process 300 is completed, the sensing process 300 may be restarted. In other embodiments, as soon as the sensing process 300 is completed, the sensing process 300 may be performed again after a period of time. The period of time may be predetermined or may be triggered by the processor 75 as needed or otherwise required.

In one embodiment, measuring a first impedance of a temperature sensing circuit (step 304) includes measuring the first impedance of a first thermistor and an RC circuit in series, e.g., by an ohmmeter in communication with and as directed by the processor 75. For example, measuring the first impedance may include measuring, e.g., with an ohmmeter such as the measurement circuitry 77, an impedance between a first sensing wire connected to the first thermistor and a second sensing wire connected to the RC circuit. In one embodiment, the measurement circuitry 77 includes the analog to digital converter and may convert the first impedance measurement into a digital signal provided to the processor 75.

In one embodiment, measuring a first impedance of a temperature sensing circuit (step 304) includes providing a first sensing signal having a first frequency to the temperature sensing circuit and measuring the impedance of the temperature sensing circuit with the first sensing signal applied thereto. In some embodiments, the first frequency may be zero, i.e., the first sensing signal may be a DC signal. When the first frequency is zero, the first impedance is a total impedance of the temperature sensing circuit.

In one embodiment, measuring a second impedance of the temperature sensing circuit (step 308) includes measuring the second impedance of the first thermistor and the RC circuit in series, e.g., by an ohmmeter in communication with and as directed by the processor 75. For example, measuring the second impedance may include measuring, e.g., with an ohmmeter such as the measurement circuitry 77, a second impedance between the first sensing wire connected to the first thermistor and the second sensing wire connected to the RC circuit. In one embodiment, the measuring circuitry 77includes an analog to digital converter, and may convert the second impedance measurement into a digital signal provided to the processor 75.

In one embodiment, measuring a second impedance of the temperature sensing circuit (step 308) includes providing a second sensing signal having a second frequency to the temperature sensing circuit and measuring the impedance of the temperature sensing circuit with the second sensing signal applied thereto. The second frequency may be between 10-100,000 or more, times, greater than the first frequency. If the first frequency is 1 kHz, the second frequency may be between 10 kHz and 100 MHz, or between 10 kHz and 1 GHz, for example.

In one embodiment, measuring the second impedance of the temperature sensing circuit (step 308) includes selecting a second frequency of the second sensing signal such that the capacitor of the RC circuit has a capacitive reactance approaching zero.

In one embodiment, determining a first temperature of the thermistor (step 312) includes determining the first temperature of the first thermistor based on the second impedance. For example, when the second sensing signal is applied to the temperature sensing circuit, the capacitor of the RC circuit has a capacitive reactance approaching zero, resulting in a impedance of the temperature sensing circuit approaching the resistance of the first thermistor. Thus, as the impedance of the temperature sensing circuit approaches the resistance of the first thermistor, when the impedance of the temperature sensing circuit is measured, the measured impedance is equivalent to the first resistance of the first thermistor. The first resistance may then be used, along with known characteristics and properties of the first thermistor relating the resistance to a temperature, to calculate the first temperature of the first thermistor.

In one embodiment, determining a second temperature of the RC circuit (step 316) includes determining the second temperature of a second thermistor of the RC circuit based on the first impedance and the second impedance. For example, as described above in more detail in relation to FIG. 4, the first impedance is a combined resistance of the first thermistor and impedance of the RC circuit and the second impedance is effectively the resistance of the first thermistor, therefore, in order to determine a resistance of the second thermistor, the second resistance (e.g., the resistance of the first thermistor) is subtracted from the first impedance (e.g., the combined resistance of the first thermistor and impedance of the RC circuit), thereby resulting in an impedance of the RC circuit. The RC circuit impedance here is equivalent to the second thermistor resistance. The second thermistor resistance may then be used, along with known characteristics and properties of the second thermistor relating the resistance to a temperature, to calculate the second temperature of the second thermistor.

In one embodiment, the sensing process 300 may be used to determine a temperature of a particular thermistor of the temperature sensing circuit, for example, a particular thermistor of the first temperature sensing circuit 200a of FIG. 5B. In this embodiment, the particular thermistor may be the thermistor of the RC circuit 112f having a particular capacitor in parallel therewith, for example.

Here, measuring the first impedance of the temperature sensing circuit (step 304) includes providing the first sensing signal having a first frequency to the temperature sensing circuit and measuring the impedance of the temperature sensing circuit with the first sensing signal applied thereto. The first frequency is selected such that when the first sensing signal is applied to the temperature sensing circuit, the capacitor of the next highest capacitance to the particular capacitor has a capacitive reactance approaching zero. In this manner, the first impedance is the combined impedance of the RC circuit having the particular thermistor with the impedance of all RC circuits having a capacitor with a capacitance less than the particular capacitor, including resistance of a thermistor 204, not wired in parallel with any capacitor, when present.

Then, measuring a second impedance of the temperature sensing circuit (step 308) includes providing the second sensing signal having a second frequency to the temperature sensing circuit and measuring the second impedance of the temperature sensing circuit with the second sensing signal applied thereto. The second frequency is selected such that, when the second sensing signal is applied to the temperature sensing circuit, the particular capacitor has a capacitive reactance approaching zero. In this manner, the second impedance, then, is the combined impedance of all RC circuits having a capacitor with a capacitance less than the particular capacitor, including resistance of a thermistor 204, not wired in parallel with any capacitor, when present.

The temperature of the particular thermistor, therefor, may be determined based upon the first impedance and the second impedance (e.g., similar to step 316). A particular resistance of the particular thermistor may be calculated by subtracting the second impedance from the first impedance. The particular resistance may then be used, along with known characteristics and properties of the particular thermistor relating the resistance to a temperature, to calculate the particular temperature of the particular thermistor.

From the above description, it is clear that the inventive concepts disclosed and claimed herein are well adapted to carry out the objects and to attain the advantages mentioned herein, as well as those inherent in the disclosure. While exemplary embodiments of the inventive concepts have been described for purposes of this disclosure, it will be understood that numerous changes may be made which will readily suggest themselves to those skilled in the art.

The foregoing description provides illustration and description, but is not intended to be exhaustive or to limit the inventive concepts to the precise form disclosed. Modifications and variations are possible in light of the above teachings or may be acquired from practice of the methodologies set forth in the present disclosure.

Even though particular combinations of features and steps are disclosed in the specification, these combinations are not intended to limit the disclosure. In fact, many of these features and steps may be combined in ways not specifically disclosed in the specification.

Similarly, although each illustrative embodiment listed above may directly depend on only one other illustrative embodiment, the disclosure includes each illustrative embodiment in combination with every other illustrative embodiment in the set of illustrative embodiments for each mode of the inventive concepts disclosed herein.

No element, act, or instruction used in the present application should be construed as critical or essential to the disclosure unless explicitly described as such outside of the preferred embodiment. Further, the phrase "based on" is intended to mean "based, at least in part, on" unless explicitly stated otherwise.

## Claims

1. A transducer array, comprising:
a first electrode (78);
a second electrode (78); and
a temperature sensing circuit (200);
wherein the temperature sensing circuit (200) comprises:
a first thermistor (204c) adjacent to the first electrode (78), the first thermistor (204c) being a first variable resistor whose resistance varies with temperature;
an RC circuit (112a) coupled in series with the first thermistor (204c), the RC circuit (112a) comprising a second thermistor (204a) adjacent to the second electrode (78) and a capacitor (208a) in parallel with the second thermistor (204a), the second thermistor (204a) being a second variable resistor whose resistance varies with temperature; and
a lead (58a, b) configured to carry an electrical signal to the electrodes (78), the lead (58a, b) further having a first sensor wire (108a) electrically coupled to the first thermistor (204c) and a second sensor wire (108b) electrically coupled to the RC circuit (112a) opposite the first thermistor (204c);
wherein the temperature sensing circuit (200) is configured such that, when a first sensing signal having a first frequency is applied to the first sensor wire (108a), the second thermistor (204a) provides an impedance to the temperature sensing circuit (200), and, when a second sensing signal having a second frequency greater than the first frequency is applied to the first sensor wire (108a), the reactance of the capacitor (208a) approaches zero and the RC circuit (112a) is shorted across the second thermistor (204a) and provides no impedance to the temperature sensing circuit (200).

2. The transducer array of claim 1, wherein the capacitor (208a) has a capacitance between 10 mf and 1 pf.

3. The transducer array of claim 1, wherein the second frequency is within a range of one order of magnitude to four orders of magnitude greater than the first frequency.

4. The transducer array of claim 1, wherein the RC circuit (112a) is a first RC circuit and the capacitor (208a) is a first capacitor, and further comprising:
a third electrode (78); and
a second RC circuit (112b) coupled in series with the first thermistor (204c) and the first RC circuit (112a), the second RC circuit (112b) comprising a third thermistor (204b) adjacent to the third electrode (78) and a second capacitor (208b) in parallel with the third thermistor (204b), the third thermistor (204b) being a third variable resistor whose resistance varies with temperature;
wherein the second frequency is insufficient to cause the reactance of the second capacitor (208b) to approach zero, and the temperature sensing circuit (200) is configured such that, when a third sensing signal having a third frequency greater than the second frequency is applied to the first sensor wire (108a), the reactances of the first and second capacitors (208a, b) approach zero and the first and second RC circuits (112a, b) are shorted across the second and third thermistors (204a, b) and provide no impedance to the temperature sensing circuit (200).

5. The transducer array of claim 4, wherein the first thermistor (204c) is in direct contact with the first electrode (78).

6. The transducer array of claim 4, wherein the third frequency is in a range from one order of magnitude to four orders of magnitude greater than the first and second frequencies.

7. The transducer array of claim 4, wherein the capacitance of the second capacitor (208b) is 100 to 10000 times greater than the capacitance of the first capacitor (208a).

8. The transducer array of claim 4, wherein the first and second capacitors (208a, b) have a capacitance between 10 mf and 1 pf.

9. The transducer array of claim 1, wherein the temperature sensing circuit (200) does not have a capacitor in parallel with the first thermistor (204c).

10. A tumor treating field system, comprising:
an electric field generator (54) configured to generate an electrical signal having an alternating current waveform at a frequency in a range from 50 kHz to 1 MHz; the transducer array (70a, b) of any of claims 1 to 9 electrically coupled to the electric field generator (54) by the lead (58a, b); and
a controller (74) in communication with the electric field generator (54), the first sensor wire (108a) and the second sensor wire (108b), the controller (74) having a processor (75) and a non-transitory computer-readable medium (76) storing computer-executable instructions that, when executed by the processor (75), are configured to cause the processor (75) to:
provide a first sensing signal along the first sensor wire (108a), the first sensing signal having a first frequency;
measure a first impedance between the first sensor wire (108a) and the second sensor wire (108b);
provide a second sensing signal along the first sensor wire (108a), the second sensing signal having a second frequency greater than the first frequency;
measure a second impedance between the first sensor wire (108a) and the second sensor wire (108b);
determine a first temperature of the first thermistor (204c) based on the second impedance; and
determine a second temperature of the second thermistor (204a) based on the first impedance and the second impedance.

11. The system of claim 10, wherein the second frequency is within a range of one order of magnitude to four orders of magnitude greater than the first frequency.

12. The system of claim 10, wherein the computer-executable instructions, when executed by the processor (75), are configured to cause the processor (75) to:
provide a third sensing signal along the first sensor wire (108a), the third sensing signal having a third frequency greater than the second frequency;
measure a third impedance between the first sensor wire (108a) and the second sensor wire (108b); and
determine a third temperature of the third thermistor (204b) based on the first impedance, the second impedance and the third impedance.

13. The system of claim 12, wherein the third frequency is in a range from one order of magnitude to four orders of magnitude greater than the first and second frequencies.

## Patentansprüche

1. Wandleranordnung, umfassend:
eine erste Elektrode (78);
eine zweite Elektrode (78); und
eine Temperaturmessschaltung (200);
wobei die Temperaturmessschaltung (200) Folgendes umfasst:
einen ersten Thermistor (204c) neben der ersten Elektrode (78), wobei der erste Thermistor (204c) ein erster variabler Widerstand ist, dessen Widerstand sich mit der Temperatur ändert; ein RC-Glied (112a), das in Reihe mit dem ersten Thermistor (204c) geschaltet ist, wobei das RC-Glied (112a) einen zweiten Thermistor (204a) neben der zweiten Elektrode (78) und einen Kondensator (208a) parallel zu dem zweiten Thermistor (204a) umfasst, wobei der zweite Thermistor (204a) ein zweiter variabler Widerstand ist, dessen Widerstand sich mit der Temperatur ändert; und
eine Leitung (58a, b), die so konfiguriert ist, dass sie ein elektrisches Signal zu den Elektroden (78) führt, wobei die Leitung (58a, b) ferner einen ersten Sensordraht (108a), der elektrisch mit dem ersten Thermistor (204c) verbunden ist, und einen zweiten Sensordraht (108b) aufweist, der elektrisch mit dem RC-Glied (112a) gegenüber dem ersten Thermistor (204c) verbunden ist;
wobei die Temperaturmessschaltung (200) so konfiguriert ist, dass beim Anlegen eines ersten Messsignals mit einer ersten Frequenz an den ersten Sensordraht (108a) der zweite Thermistor (204a) eine Impedanz für die Temperaturmessschaltung (200) bildet und beim Anlegen eines zweiten Messsignals mit einer zweiten Frequenz, die größer als die erste Frequenz ist, an den ersten Sensordraht (108a) die Reaktanz des Kondensators (208a) gegen Null strebt und das RC-Glied (112a) über den zweiten Thermistor (204a) kurzgeschlossen wird und keine Impedanz für die Temperaturmessschaltung (200) bildet.

2. Wandleranordnung nach Anspruch 1, wobei der Kondensator (208a) eine Kapazität zwischen 10 mF und 1 pF aufweist.

3. Wandleranordnung nach Anspruch 1, wobei die zweite Frequenz in einem Bereich liegt, der um eine Größenordnung bis vier Größenordnungen größer ist als die erste Frequenz.

4. Wandleranordnung nach Anspruch 1, wobei das RC-Glied (112a) ein erstes RC-Glied ist und der Kondensator (208a) ein erster Kondensator ist, und weiter umfassend:
eine dritte Elektrode (78); und
ein zweites RC-Glied (112b), der in Reihe mit dem ersten Thermistor (204c) und dem ersten RC-Glied (112a) geschaltet ist, wobei das zweite RC-Glied (112b) einen dritten Thermistor (204b) neben der dritten Elektrode (78) und einen zweiten Kondensator (208b) parallel zu dem dritten Thermistor (204b) umfasst, wobei der dritte Thermistor (204b) ein dritter variabler Widerstand ist, dessen Widerstand sich mit der Temperatur ändert;
wobei die zweite Frequenz nicht ausreicht, um die Reaktanz des zweiten Kondensators (208b) gegen Null zu treiben, und die Temperaturmessschaltung (200) so konfiguriert ist, dass beim Anlegen eines dritten Messsignals mit einer dritten Frequenz, die größer als die zweite Frequenz ist, an den ersten Sensordraht (108a) die Reaktanzen des ersten und des zweiten Kondensators (208a, b) gegen Null streben und das erste und das zweite RC-Glied (112a, b) über den zweiten und den dritten Thermistor (204a, b) kurzgeschlossen werden und keine Impedanz für die Temperaturmessschaltung (200) bieten.

5. Wandleranordnung nach Anspruch 4, wobei der erste Thermistor (204c) in direktem Kontakt mit der ersten Elektrode (78) steht.

6. Wandleranordnung nach Anspruch 4, wobei die dritte Frequenz in einem Bereich liegt, der um eine Größenordnung bis vier Größenordnungen größer ist als die erste und die zweite Frequenz.

7. Wandleranordnung nach Anspruch 4, wobei die Kapazität des zweiten Kondensators (208b) 100- bis 10000-mal größer ist als die Kapazität des ersten Kondensators (208a).

8. Wandleranordnung nach Anspruch 4, wobei der erste und der zweiten Kondensator (208a, b) eine Kapazität zwischen 10 mf und 1 pF aufweisen.

9. Wandleranordnung nach Anspruch 1, wobei der Temperaturmesskreis (200) keinen Kondensator parallel zu dem ersten Thermistor (204c) aufweist.

10. Tumor-Treating-Field-System umfassend:
einen elektrischen Feldgenerator (54), der konfiguriert ist, um ein elektrisches Signal mit einer Wechselstromwellenform bei einer Frequenz im Bereich von 50 kHz bis 1 MHz zu erzeugen; und
die Wandleranordnung (70a, b) nach einem der Ansprüche 1 bis 9, die über die Leitung (58a, b) elektrisch mit dem elektrischen Feldgenerator (54) gekoppelt ist; und
eine Steuereinheit (74) in Kommunikation mit dem elektrischen Feldgenerator (54), dem ersten Sensordraht (108a) und dem zweiten Sensordraht (108b), wobei die Steuereinheit (74) einen Prozessor (75) und ein nichtflüchtiges computerlesbares Medium (76) aufweist, das computerausführbare Anweisungen speichert, die, wenn sie von dem Prozessor (75) ausgeführt werden, so konfiguriert sind, dass sie den Prozessor (75) zu Folgendem veranlassen:
Bereitstellen eines ersten Messsignals entlang des ersten Sensordrahtes (108a), wobei das erste Messsignal eine erste Frequenz aufweist;
Messen einer ersten Impedanz zwischen dem ersten Sensordraht (108a) und dem zweiten Sensordraht (108b);
Bereitstellen eines zweiten Messsignals entlang des ersten Sensordrahtes (108a), wobei das zweite Messsignal eine zweite Frequenz aufweist, die größer als die erste Frequenz ist;
Messen einer zweiten Impedanz zwischen dem ersten Sensordraht (108a) und dem zweiten Sensordraht (108b);
Bestimmen einer ersten Temperatur des ersten Thermistors (204c) anhand der zweiten Impedanz; und
Bestimmen einer zweiten Temperatur des zweiten Thermistors (204a) anhand der ersten Impedanz und der zweiten Impedanz.

11. System nach Anspruch 10, wobei die zweite Frequenz in einem Bereich liegt, der um eine Größenordnung bis vier Größenordnungen größer ist als die erste Frequenz.

12. System nach Anspruch 10, wobei die computerausführbaren Anweisungen, wenn sie von dem Prozessor (75) ausgeführt werden, so konfiguriert sind, dass sie den Prozessor (75) zu Folgendem veranlassen:
Bereitstellen eines dritten Messsignals entlang des ersten Sensordrahtes (108a), wobei das dritte Messsignal eine dritte Frequenz aufweist, die größer als die zweite Frequenz ist;
Messen einer dritten Impedanz zwischen dem ersten Sensordraht (108a) und dem zweiten Sensordraht (108b);
Bestimmen einer dritten Temperatur des dritten Thermistors (204b) anhand der ersten Impedanz, der zweiten Impedanz und der dritten Impedanz.

13. System nach Anspruch 12, wobei die dritte Frequenz in einem Bereich liegt, der um eine Größenordnung bis vier Größenordnungen größer ist als die erste und die zweite Frequenz.

## Revendications

1. Réseau de transducteurs, comprenant :
une première électrode (78) ;
une deuxième électrode (78) ; et
un circuit de détection de température (200) ;
dans lequel le circuit de détection de température (200) comprend :
une première thermistance (204c) adjacente à la première électrode (78), la première thermistance (204c) étant une première résistance variable dont la résistance varie avec la température ; un circuit RC (112a) couplé en série avec la première thermistance (204c), le circuit RC (112a) comprenant une deuxième thermistance (204a) adjacente à la deuxième électrode (78) et un condensateur (208a) en parallèle avec la deuxième thermistance (204a), la deuxième thermistance (204a) étant une deuxième résistance variable dont la résistance varie avec la température ; et
une liaison (58a, b) configurée pour transporter un signal électrique vers les électrodes (78), la liaison (58a, b) présentant en outre un premier fil de capteur (108a) couplé électriquement à la première thermistance (204c) et un deuxième fil de capteur (108b) couplé électriquement au circuit RC (112a) opposé à la première thermistance (204c) ;
dans lequel le circuit de détection de température (200) est configuré de telle sorte que, lorsqu'un premier signal de détection présentant une première fréquence est appliqué au premier fil de capteur (108a), la deuxième thermistance (204a) fournit une impédance au circuit de détection de température (200), et, lorsqu'un deuxième signal de détection présentant une deuxième fréquence supérieure à la première fréquence est appliqué au premier fil de capteur (108a), la réactance du condensateur (208a) tend vers zéro et le circuit RC (112a) est court-circuité à travers la deuxième thermistance (204a) et ne fournit aucune impédance au circuit de détection de température (200).

2. Réseau de transducteurs de la revendication 1, dans lequel le condensateur (208a) présente une capacitance comprise entre 10 mf et 1 pf.

3. Réseau de transducteurs selon la revendication 1, dans lequel la deuxième fréquence se situe dans une plage allant d'un ordre de grandeur à quatre ordres de grandeur supérieurs à la première fréquence.

4. Réseau de transducteurs selon la revendication 1, dans lequel le circuit RC (112a) est un premier circuit RC et le condensateur (208a) est un premier condensateur, et comprenant en outre :
une troisième électrode (78) ; et
un second circuit RC (112b) couplé en série avec la première thermistance (204c) et le premier circuit RC (112a), le second circuit RC (112b) comprenant une troisième thermistance (204b) adjacente à la troisième électrode (78) et un second condensateur (208b) en parallèle avec la troisième thermistance (204b), la troisième thermistance (204b) étant une troisième résistance variable dont la résistance varie avec la température ;
dans lequel la deuxième fréquence est insuffisante pour amener la réactance du second condensateur (208b) à tendre vers zéro, et le circuit de détection de température (200) est configuré de telle sorte que, lorsqu'un troisième signal de détection présentant une troisième fréquence supérieure à la deuxième fréquence est appliqué au premier fil de capteur (108a), les réactances des premier et second condensateurs (208a, b) tendent vers zéro, et les premier et second circuits RC (112a, b) sont court-circuités à travers les deuxième et troisième thermistances (204a, b) et ne fournissent aucune impédance au circuit de détection de température (200).

5. Réseau de transducteurs selon la revendication 4, dans lequel la première thermistance (204c) est en contact direct avec la première électrode (78).

6. Réseau de transducteurs selon la revendication 4, dans lequel la troisième fréquence se situe dans une plage allant d'un ordre de grandeur à quatre ordres de grandeur supérieurs aux première et deuxième fréquences.

7. Réseau de transducteurs de la revendication 4, dans lequel la capacitance du second condensateur (208b) est de 100 à 10 000 fois supérieure à la capacitance du premier condensateur (208a).

8. Réseau de transducteurs selon la revendication 4, dans lequel les premier et second condensateurs (208a, b) présentent une capacitance comprise entre 10 mf et 1 pf.

9. Réseau de transducteurs selon la revendication 1, dans lequel le circuit de détection de température (200) ne présente pas de condensateur en parallèle avec la première thermistance (204c).

10. Système de champ de traitement de tumeur, comprenant :
un générateur de champ électrique (54) configuré pour fournir un premier signal électrique présentant une première forme d'onde de courant alternatif à une fréquence située dans une plage allant de 50 kHz à 1 MHz ;
le réseau de transducteurs (70a, b) selon l'une quelconque des revendications 1 à 9 couplé électriquement au générateur de champ électrique (54) par la liaison (58a, b) ; et
un contrôleur (74) en communication avec le générateur de champ électrique (54), le premier fil de capteur (108a) et le second fil de capteur (108b), le contrôleur (74) présentant un processeur (75) et un support non transitoire lisible par ordinateur (76) stockant des instructions exécutables par ordinateur qui, lorsqu'elles sont exécutées par le processeur (75), sont configurées pour amener le processeur (75) à :
fournir un premier signal de détection le long du premier fil de capteur (108a), le premier signal de détection présentant une première fréquence ;
mesurer une première impédance entre le premier fil de capteur (108a) et le second fil de capteur (108b) ;
fournir un deuxième signal de détection le long du premier fil de capteur (108a), le deuxième signal de détection présentant une deuxième fréquence supérieure à la première fréquence ;
mesurer une deuxième impédance entre le premier fil de capteur (108a) et le second fil de capteur (108b) ;
déterminer une première température de la première thermistance (204c) sur la base de la deuxième impédance ; et
déterminer une deuxième température de la deuxième thermistance (204a) sur la base de la première impédance et de la deuxième impédance.

11. Système selon la revendication 10, dans lequel la seconde fréquence se situe dans une plage allant d'un ordre de grandeur à quatre ordres de grandeur supérieurs à la première fréquence.

12. Système selon la revendication 10, dans lequel les instructions exécutables par ordinateur, lorsqu'elles sont exécutées par le processeur (75), sont configurées pour amener le processeur (75) à :
fournir un troisième signal de détection le long du premier fil de capteur (108a), le troisième signal de détection présentant une troisième fréquence supérieure à la deuxième fréquence ;
mesurer une troisième impédance entre le premier fil de capteur (108a) et le second fil de capteur (108b) ; et
déterminer une troisième température de la troisième thermistance (204b) sur la base de la première impédance, de la deuxième impédance et de la troisième impédance.

13. Système selon la revendication 12, dans lequel la troisième fréquence se situe dans une plage allant d'un ordre de grandeur à quatre ordres de grandeur supérieurs aux première et deuxième fréquences.
